# EUROPEAN PATENT APPLICATION

(11) **EP 4 610 989 A1**
(43) Date of publication of application: **03.09.2025**
(21) Application number: 23882557.4
(22) Date of filing: 20.10.2023
(51) Int. Cl.: G16H 10/00, A61B 5/352, G06Q 50/10, G06Q 50/22

(54) **INFORMATION PROCESSING DEVICE, INFORMATION PROCESSING METHOD, AND PROGRAM**

(30) Priority: 24.10.2022 JP 2022169966
(71) Applicant: Suntory Holdings Limited, Osaka 530-8203 (JP)
(72) Inventor: MIZUTANI, Haruo, Kyoto 6190284 (JP)
(74) Representative: Vossius & Partner Patentanwälte Rechtsanwälte mbB
(86) International application number: PCT/JP2023/038002
(87) International publication number: WO 2024/090352

(57) **Abstract**

Since it is useful if information on a status of a living body of a user can be utilized, an information processing apparatus 100 includes: a storage unit 110 in which information is stored; a biological information acquiring unit 143 that acquires measurement information on a status of a living body of a providing user; a content generating unit 161 that generates content information using the measurement information; and a content accumulating unit 163 that accumulates the content information generated by the content generating unit 161, in the storage unit 110, in association with a provider identifier with which the providing user can be identified. Accordingly, it is possible to generate content information from information on a status of a living body of a providing user, and to utilize the information on the status of the living body of the user.

## Description

### Technical Field

The present invention relates to an information processing apparatus, an information processing method, and a program that are capable of acquiring information on a status of a living body of a user.

### Background Art

In recent years, users have become increasingly aware of their own health, and various devices and services have been provided to assist users in understanding their health status. For example, Patent Document 1 below discloses a system configured to determine a health risk signal of a user based on an aggregate heartbeat value determined according to biological measurement data obtained from the user, and to provide the signal to the user.

Furthermore, Patent Document 2 discloses a relaxation level determination apparatus that can easily and accurately determine the relaxation level by using the heartbeat (pulse) signal of an examinee who is a user.

It is known that various rhythms affect human sleeping and waking. Such rhythms include, for example, a rhythm called circadian rhythm (circadian cycle). The so-called two-process rhythm is also widely known as the human sleep-wake rhythm (see Non-Patent Document 1 below, for example).

Furthermore, in addition to the conventionally known Transformer, a method called Informer has been proposed as a machine learning method that can be used for prediction of future time-series data, as described in Non-Patent Document 2 below.

### Citation List

### Patent Documents

Patent Document 1: Japanese Patent No. 6531161
Patent Document 2: JP H9-70399A

### Non-Patent Documents

Non-Patent Document 1: Daan S, Beersma DG, Borbely AA. Timing of human sleep: recovery process gated by a circadian pacemaker. Am J Physiol. 1984; 246 (2 Pt 2): R161-83.
Non-Patent Document 2: Zhou, Haoyi, et al. "Informer: Beyond efficient transformer for long sequence time series forecasting." Proceedings of the AAAI Conference on Artificial Intelligence. Vol. 35. No. 12.

### Summary of Invention

### Technical Problem

Incidentally, conventionally, information on a status of a living body of a user is usually used mainly to acquire information on a health status of that user or to make a diagnosis of that user. It will be useful if such information on the status of a living body can be utilized for other purposes as well.

It is an object of the present invention to provide an information processing apparatus, an information processing method, and a program that are capable of utilizing information on a status of a living body of a user.

### Solution to Problem

A first aspect of the present invention is directed to an information processing apparatus including: a biological information acquiring unit that acquires measurement information on a status of a living body of a providing user; a content generating unit that generates content information using the measurement information; and a content accumulating unit that accumulates the content information generated by the content generating unit, in a storage unit in which information is stored.

With this configuration, it is possible to generate content information from information on a status of a living body of a providing user.

Furthermore, a second aspect of the present invention is directed to the information processing apparatus according to the first aspect, wherein the content generating unit includes a specifying information acquiring unit that acquires specifying information for specifying a status of the providing user, using the measurement information, and is configured to acquire the content information based on the specifying information.

With this configuration, it is possible to easily generate content information according to a status of a providing user.

Furthermore, a third aspect of the present invention is directed to the information processing apparatus according to the second aspect, wherein the specifying information contains degree information indicating a degree to which the status of the providing user falls under a predetermined category.

With this configuration, it is possible to more reliably generate content information according to a status of a providing user.

Furthermore, a fourth aspect of the present invention is directed to the information processing apparatus according to any one of the first to third aspects, wherein the content generating unit generates the content information using learning information configured such that input information containing the measurement information or information based on the measurement information is used to output output information.

With this configuration, it is possible to easily generate content information according to information on a status of a living body of a providing user.

Furthermore, a fifth aspect of the present invention is directed to the information processing apparatus according to any one of the first to fourth aspects, further including: a token recording unit that performs recording processing that records a non-fungible token corresponding to the content information generated by the content generating unit, in a predetermined manner in association with an owner identifier for identifying an owning user of the content information; and an owner processing unit that performs predetermined owner processing using the owner identifier corresponding to the token in a case in which a predetermined processing condition corresponding to the token is satisfied.

With this configuration, it is possible to reliably perform owner processing for an owning user of content information.

Furthermore, a sixth aspect of the present invention is directed to the information processing apparatus according to the fifth aspect, wherein in a case in which the token recording unit acquires sales information on a sale of the content information, the token recording unit performs the recording processing such that the token corresponding to the sold content information is associated with the owner identifier corresponding to the sales information.

With this configuration, it is possible to record information with which an owning user of content information can be reliably specified, according to a sale of the content information.

Furthermore, a seventh aspect of the present invention is directed to the information processing apparatus according to the fifth or sixth aspect, wherein the token recording unit is configured to perform recording processing that records the token in a predetermined manner in association with a provider identifier for identifying a providing user corresponding to the content information, and the information processing apparatus further includes a provider processing unit that performs predetermined provider processing using the provider identifier corresponding to the token in a case in which a predetermined processing condition corresponding to the token is satisfied.

With this configuration, it is possible to perform provider processing for a providing user of content information.

Furthermore, an eighth aspect of the present invention is directed to the information processing apparatus according to the seventh aspect, wherein the provider processing unit grants a predetermined reward to the providing user using the provider identifier corresponding to the token, in a case in which the token recording unit performs recording processing that records the token newly in association with the owner identifier.

With this configuration, it is possible to grant a reward to a providing user in a case in which owner information corresponding to content information is newly recorded.

### Advantageous Effects of Invention

With the information processing apparatus and the like according to the present invention, it is possible to generate content information from information on a status of a living body of a providing user, and to utilize the information on the status of the living body of the user.

### Brief Description of Drawings

FIG. 1 is a diagram showing the outline of an information processing system according to an embodiment of the present invention.
FIG. 2 is a block diagram of an information processing apparatus in the embodiment.
FIG. 3 is a diagram showing an example of content information stored in a content information storage unit in the embodiment.
FIG. 4 is a block diagram of a terminal device and a measuring device in the embodiment.
FIG. 5 is a first flowchart showing an example of an operation of the information processing apparatus in the embodiment.
FIG. 6 is a second flowchart showing an example of an operation of the information processing apparatus in the embodiment.
FIG. 7 is a flowchart showing an example of content generating processing of the information processing apparatus in the embodiment.
FIG. 8 is a diagram illustrating measurement information in a specific example of this embodiment.
FIG. 9 is a diagram showing an example of measurement information before filter processing in a specific example of this embodiment.
FIG. 10 is a diagram showing an example of measurement information after filter processing in a specific example of this embodiment.
FIG. 11 is a diagram illustrating an intermediate representation system representing specifying information in a specific example of this embodiment.
FIG. 12 is a diagram showing an example of the generation of content information in a specific example of this embodiment.
FIG. 13 is a diagram illustrating an example of the use of the information processing system in the embodiment.
FIG. 14 is a schematic view of a computer system in the foregoing embodiment.
FIG. 15 is a block diagram of the computer system in the embodiment.

### Description of Embodiment

Hereinafter, an embodiment of an information processing apparatus and the like will be described with reference to the drawings. It should be noted that constituent elements denoted by the same reference numerals in the embodiments perform similar operations, and thus a description thereof may not be repeated.

The terms used below are generally defined as follows. The meanings of these terms do not always have to be interpreted as indicated herein, and have to be interpreted in light of individual explanations below, if any, for example.

"Content information" is information for representing content that can be viewed and understood by a user. The content is, for example, images (which may be still or moving images), sounds (voice, music, etc.), tones, colors, sentences, poems, drawings, shapes (which may be three-dimensional or two-dimensional shapes), or the like. The content information may be information that represents these types of content as they are, or it may be information specific to these types of content for use in outputting the content by executing predetermined processing or the like using a computer or a predetermined device or the like. The content information may be information that specifies the location of the content.

"Providing user" is a user who provides information that is used to generate the content information. The providing user may also be said to be a provider. "Owning user" is a user who possesses the generated content information. The owning user may also be said to be a user who owns content. The owning user may also be said to be an owner or a possessor.

"Identifier for a matter" is a letter, a symbol, or the like for uniquely identifying the matter. The identifier is an ID, for example, but may be any type of information with which the corresponding matter can be identified. That is to say, the identifier may be the name of the matter itself that it indicates, or symbols that are combined so as to uniquely correspond to the matter.

"Acquiring" may include acquiring a matter that is input by a user or the like, or acquiring information stored in the apparatus or another apparatus (the information may be information stored in advance or information generated through information processing in the apparatus). "Acquiring information stored in another apparatus" may include acquiring information stored in the other apparatus via an API or the like, or acquiring the content of a document file (including the webpage content, etc.) provided by the other apparatus.

Furthermore, a so-called machine learning method may be used to acquire information. A machine learning method may be used as follows, for example. That is to say, a learning model (learning information) in which a particular type of input information is taken as input and a type of output information that is to be acquired is taken as output is configured using a machine learning method. For example, two or more pairs of input information and output information are prepared in advance, the two or more pairs of information are given to a module for configuring a learning model of machine learning to configure a learning model, and the configured learning model is accumulated in a storage unit. The learning model may also be said to be a classifier. There is no limitation on the machine learning method, and examples thereof include deep learning, random forests, and SVM. For example, functions in various machine learning frameworks and various existing libraries, such as fastText, tinySVM, random forest, and TensorFlow, can be used for the machine learning. The acquiring information using such a learning model may also be said to be acquiring information through machine learning.

"Learning model" is not limited to those obtained through machine learning. The learning model may be a table indicating a correspondence relationship between an input vector based on input information or the like and output information, for example. In this case, output information corresponding to a feature vector based on input information may be acquired from the table, or a vector that is close to a feature vector based on the input information may be generated using two or more input vectors in the table and parameters for weighting the input vectors, and output information corresponding to the input vectors and parameters used for the generation may be used to acquire final output information. The acquiring information using such a learning model may also be said to be acquiring information using a correspondence relationship. The learning model may be a function or the like that represents a relationship between an input vector based on the input information or the like and information for generating output information, for example. In this case, for example, information corresponding to a feature vector based on input information may be obtained using a function, and the obtained information may be used to acquire output information. The acquiring information using such a learning model may also be said to be acquiring information using a function.

"Outputting information" is a concept that encompasses display on a display screen, projection using a projector, printing by a printer, output of a sound, transmission to an external apparatus, accumulation in a recording medium, and delivery of a processing result to another processing apparatus or another program. Specifically, for example, this concept encompasses enabling information to be displayed on a webpage, transmission as an email or the like, and outputting information for printing.

"Accepting information" is a concept that encompasses accepting information input from an input device such as a keyboard, a mouse, or a touch panel, receiving information transmitted from another apparatus or the like via a wired or wireless communication line, and accepting information read from a recording medium such as an optical disk, a magnetic disk, or a semiconductor memory.

### Embodiment

This embodiment is summarized as follow. The information processing apparatus is configured to be able to acquire content information based on measurement information such as a bioelectric potential or the like of a providing user. The content information is acquired, for example, by specifying a status of the providing user based on the measurement information, and performing the acquisition using information indicating the specified status. The information indicating the specified status may contain information indicating a degree of falling under that status. The processing that specifies a status of the providing user from the measurement information and that generates content information using information indicating the status can be performed, for example, through acquisition using the above-described machine learning, acquisition using a function, or acquisition using a table. In addition, these types of processing may be performed using other predetermined rules.

Furthermore, the information processing apparatus may acquire a token corresponding to the content information, and record the token in association with owner information of the content information. If the content information is sold, the owner information of the corresponding token may be changed accordingly. Then, predetermined processing may be performed based on the owner information corresponding to the token. Examples of the predetermined processing include, but are not limited to, determination as to whether or not to refuse the provision of a service, transmission of information, and the like. The information processing apparatus may record the token corresponding to the content information, in association with provider information. Then, predetermined processing may be performed based on the provider information corresponding to the token. Examples of the predetermined processing include, but are not limited to, distribution of newly generated value of the content information, granting of authority to the providing user, transmission of information, and the like. Also, if the owner information of the token is changed, predetermined processing may be performed based on the provider information. Such owner information and provider information may be a user identifier for identifying a user, but there is no limitation to this.

In the following description, a storage unit provided in an information processing apparatus 1 may be used as the recording destination for a token and owner information or provider information associated therewith, but the recording destination is not limited to this. For example, the information may be recorded in a blockchain infrastructure that is accessible by the information processing apparatus 1. In other words, the information such as a token may be recorded in a distributed ledger that is accessible by the information processing apparatus 1.

Hereinafter, an example of the information processing system using the thus configured information processing apparatus will be described.

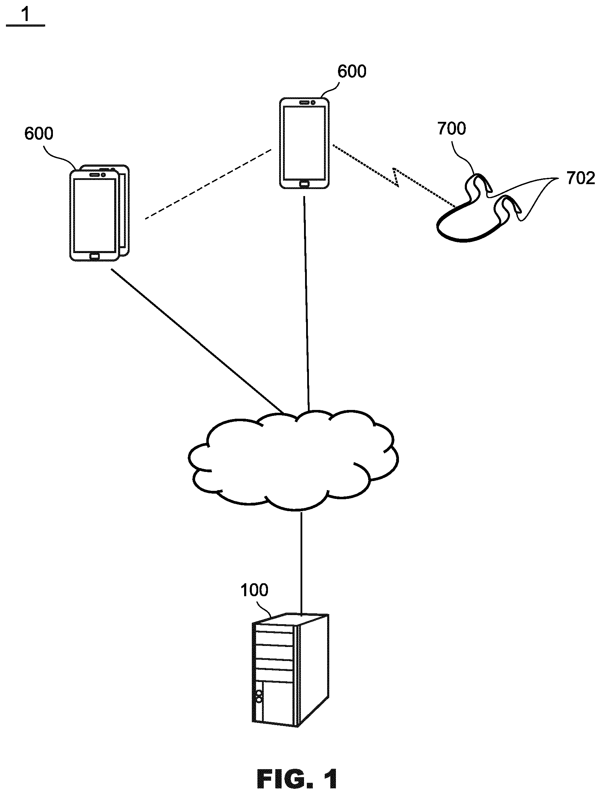

FIG. 1 is a diagram showing the outline of an information processing system 1 according to an embodiment of the present invention.

In this embodiment, the information processing system 1 includes the information processing apparatus 100, terminal devices 600, and measuring devices 700. The information processing apparatus 100 and the terminal devices 600 can communicate with each other, for example, via a network such as a local area network or the Internet. A measuring device 700 and a terminal device 600 can communicate with each other. The configuration of the information processing system 1 is not limited to this. There is no limitation on the number of apparatuses of each type included in the information processing system 1, and other types of apparatuses may also be included in the information processing system 1.

Each measuring device 700 is, for example, a so-called wearable device. The measuring device 700 has electrode units 702 that are in contact with a body surface of the user. The measuring device 700 is configured to be able to measure biological signals related to bioelectricity such as action potentials and resting potentials of the body of the user via the electrode units 702. In this embodiment, the measuring device 700 is configured to be able to measure time-series biological signals such as brain waves and pulse waves.

The measuring device 700 may be configured to be able to measure the body temperature or the like of the user by means of the electrode units 702 or other unshown units. The measuring device 700 may include a measuring unit for measuring biological signals of the user instead of the electrode units 702 or in addition to the electrode units 702. The measuring unit may be configured to be able to detect a target different from the bioelectricity. For example, the measuring unit may be able to receive light emitted or reflected from the body of the user to obtain time-series measurement results. That is to say, the measuring device 700 may be configured, for example, to be able to receive light emitted or reflected from the body of the user and to measure information on the body of the user based on the results thereof. The measuring device 700 includes, for example, a storage unit (not shown) in which measurement results, control programs for the measuring device 700, and the like are stored, a processing unit (not shown) that performs various processing operations using the information stored in the storage unit, a transmitting and receiving unit (not shown) for transmitting and receiving information, and the like.

The measuring device 700 is, for example, an ear-mounted headset that can be worn by the user at all times. The measuring device 700 is configured such that, when worn, two electrode units 702 are in contact with the vicinity of the left and right mastoid processes of the user, thereby enabling measurement of the brain waves and the pulse waves of the user. Such a configuration of the measuring device 700 is known, and thus a detailed description thereof has been omitted. The measuring device 700 is not limited to a headset type, and may be in various forms that allow the user to continue wearing it, such as eyeglasses, wristwatch, ring, neck-hanging, belt, and clothing types. The measuring device 700 may include a sound output unit (not shown) that outputs a user-hearable sound by vibrating air or an object, a display screen (not shown) that can be viewed by the user, and the like. The measuring device 700 may be configured to be able to output information to the user by means of the sound output unit, the display screen, and the like. The measuring device 700 may be two or more physically separated apparatuses that can cooperate to measure one or more biological signals. The measuring device 700 may include electrode units 702 that are used in contact with the body surface of the user only during the measurement. The measuring device 700 is not limited to such wearable devices, but may also be those that are used by the user to measure biological signals under certain circumstances, such as, for example, a blood pressure monitor, a thermometer, a scale, an electroencephalograph, or an electrocardiograph.

In this embodiment, the measurement results of time-series biological signals and information based thereon are referred to as measurement information on a status of a living body. The measurement information may be, for example, raw data of measurement results acquired by measuring biological signals, or information obtained by converting or processing the raw data. In the following description, the measurement information is information obtained by measuring a value related to a human bioelectric potential. The measurement information may also be said to be time-series information that is time-series information on a status of a living body based on measurement results of a biological signal of a user. That is to say, time-series information is measurement results of time-series biological signals, or time-series information acquired based thereon.

The measurement information is not limited to this. That is to say, it is sufficient that the information processing apparatus 100 is configured to use measurement information on bioelectric potentials measured by the measuring device 700. In other words, the measurement information is not necessarily limited to what is referred to as time-series information. In this embodiment, information obtained by measuring a user at two or more points in time, and more preferably information obtained by measuring a user in a time series is used as the measurement information.

In this embodiment, examples of the measurement results of a biological signal include, but are not limited to, ECG (electrocardiogram), pulse waves such as PPG (photoplethysmogram), brain waves, and the like.

The measuring device 700 in this embodiment is connected wirelessly or via a wired connection to the terminal device 600, and is configured to be able to transmit and receive information to and from the terminal device 600. For example, the measuring device 700 is configured to be able to perform short-range wireless communication with the terminal device 600. The measuring device 700 is configured, for example, to be able to transmit the measurement results to the terminal device 600. The measurement results can be transmitted to the terminal device 600 as time-series information accumulated by the measuring device 700, or can be transmitted sequentially to the terminal device 600. The terminal device 600 is configured to be able to perform various types of processing using information received from the measuring device 700, and to transmit the received information or information obtained by processing the received information to the information processing apparatus 100. The measuring device 700 itself may be able to be connected to a network such as a local area network or the Internet, and to communicate with the information processing apparatus 100 and the terminal device 600 connected to the network. The measurement results may be transmitted to the information processing apparatus 100. The measuring device 700 may be configured to be able to perform operations such as measuring biological signals or performing predetermined processing on measurement results by itself, or may be configured to be able to perform these operations in conjunction with the terminal device 600 by transmitting and receiving signals.

The users of the information processing system 1 can use the information processing system 1 by using the terminal devices 600 and the measuring devices 700. In FIG. 1, for example, portable information terminal devices such as so-called smartphones are shown as the terminal devices 600, but those that are used as the terminal devices 600 are not limited to such portable information terminal devices. For example, personal computers (PC) such as laptop computers may be used as the terminal devices 600, or other devices such as tablet-type information terminal devices may be used. In the examples below, a description will be given assuming that portable information terminal devices such as smartphones are used as the terminal devices 600, but there is no limitation to this.

The terminal devices 600 may include built-in measuring devices 700.

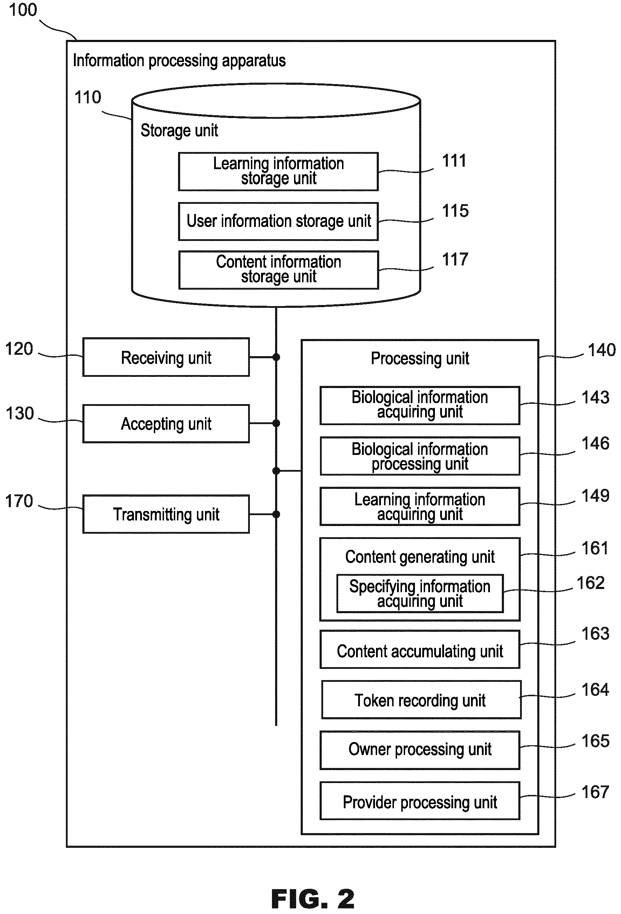

FIG. 2 is a block diagram of the information processing apparatus 100.

As shown in FIG. 2, the information processing apparatus 100 includes a storage unit 110, a receiving unit 120, an accepting unit 130, a processing unit 140, and a transmitting unit 170. The information processing apparatus 100 is, for example, a server apparatus.

The storage unit 110 includes a learning information storage unit 111, a user information storage unit 115, and a content information storage unit 117.

Learning information acquired in advance is stored in the learning information storage unit 111. In this embodiment, the learning information is generated using a so-called machine learning method. The learning information is, for example, generated and stored in the learning information storage unit 111 by the processing unit 140 as described later, but there is no limitation to this. That is to say, learning information generated in an apparatus different from the information processing apparatus 100 may be stored in the learning information storage unit 111. Details of the learning information will be described later.

In this embodiment, learning information that can be commonly used for two or more users is prepared. However, there is no limitation to this, and the learning information may be configured for each user targeted for biological signal measurement, or learning information corresponding to each user may be generated and stored in the learning information storage unit 111. In this case, each piece of learning information may be stored in association with a user identifier for identifying a user. Learning information may be prepared for each group of users who share common attributes. In this case, for example, learning information may be stored in association with an identifier for identifying a group.

User information is stored in the user information storage unit 115. In this embodiment, the user information is information in which a user identifier, which is an identifier for identifying a user who uses the information processing system 1, is associated with information on the user. The user information may contain various types of information. For example, the user information may contain information transmitted from the terminal device 600 that is used by the user, information on the user acquired by the information processing apparatus 100 as described later, and the like. The information transmitted from the terminal device 600 that is used by the user corresponds to, for example, measurement information, information input by the user to the terminal device 600, and the like. The information on the user acquired by the information processing apparatus 100 corresponds to, for example, prediction information and the like as will be described later. User information transmitted from other external apparatuses or the like may be stored in the user information storage unit 115.

Measurement information transmitted from the terminal devices 600 of the respective users is stored in the user information storage unit 115. The measurement information is, for example, measurement results obtained by the users performing measurement using the measuring devices 700, and is information stored in terminal storage units 610 of the terminal devices 600. The procedure in which the measurement information is stored in the user information storage unit 115 is not limited to this. The configuration is also possible in which measurement information prepared in advance is stored in the user information storage unit 115 and that measurement information is used.

Content information generated by the information processing apparatus 100 is stored in the content information storage unit 117. In this embodiment, the content information is stored, for example, in association with a non-fungible token corresponding to the content information. The token may be an identifier for identifying the content information. The number of tokens corresponding to one piece of content information may be only one, or two or more.

In this embodiment, a provider identifier with which a providing user corresponding to the content information can be identified and an owner identifier with which an owning user who owns the content information can be identified may be further stored in the content information storage unit 117 in association with the content information. The provider identifier may also be said to be provider information, which is user information on a provider. The owner identifier may also be said to be owner information, which is user information on an owner.

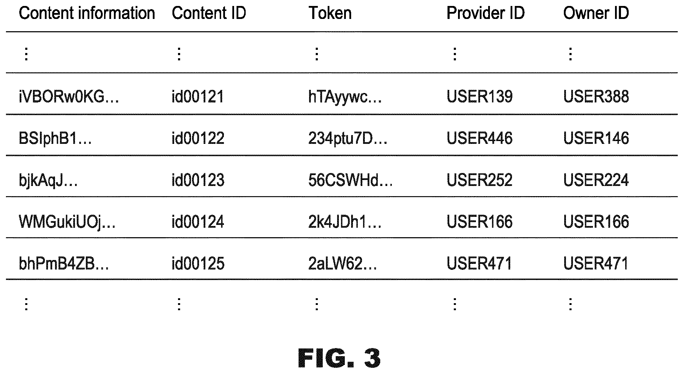

FIG. 3 is a diagram showing an example of content information stored in the content information storage unit 117.

As shown in the drawing, for example, content information is recorded in the content information storage unit 117 in association with a content identifier (content ID) for identifying the content information, a token, a provider identifier, and an owner identifier. The token is, for example, a unique character string or code string. The content information is, for example, information indicating the content itself, but it may also be recorded as, for example, a path or file name of a file that is the content. In this case, the content identifier may also be said to be stored as the content information in the content information storage unit 117. Also, the content identifier may also be said to be stored as the token in the content information storage unit 117.

Returning to FIG. 2, the receiving unit 120 receives information transmitted from other apparatuses. The receiving unit 120 stores the received information, for example, in the storage unit 110. In this embodiment, for example, the users perform input of information and the like and transmit the information to the information processing apparatus 100, using the terminal devices 600. The receiving unit 120 can store each piece of transmitted information in the storage unit 110 in association with the user identifier. In this embodiment, the receiving unit 120 receives the measurement information transmitted from the terminal devices 600, and stores the information in the storage unit 110 in association with the user identifiers. In the case in which the receiving unit 120 receives these pieces of information from the terminal devices 600, the receiving unit 120 can specify user identifiers of the users pertaining to the transmission based on the transmitted information.

The accepting unit 130 accepts information input using an unshown input part connected to the information processing apparatus 100. The accepting unit 130 stores the accepted information, for example, in the storage unit 110. The information may be input by any input part such as a numeric keypad, a keyboard, a mouse, or a menu screen. The accepting unit 130 may accept information input through an input operation performed using a reading device (e.g., code reader, etc.) connected to the information processing apparatus 100 (e.g., including information read by the device).

The accepting unit 130 may be considered to accept information received by the receiving unit 120, as information input to the information processing apparatus 100. That is to say, the input of information to the information processing apparatus 100 may be construed to mean that the information is indirectly input to the information processing apparatus 100 by the user via the terminal device 600 or the like or that the information is directly input to the information processing apparatus 100 by the user using an input part. The processing in which the user causes the information processing apparatus 100 to execute a program for automatically generating information or to function by giving various types of information to a program, thereby causing information to be given to the information processing apparatus 100 may be considered as the input of information to the information processing apparatus 100.

The processing unit 140 includes a biological information acquiring unit 143, a biological information processing unit 146, a learning information acquiring unit 149, a content generating unit 161, a content accumulating unit 163, a token recording unit 164, an owner processing unit 165, and a provider processing unit 167. In this embodiment, the content generating unit 161 includes a specifying information acquiring unit 162. The processing unit 140 performs various types of processing. The various types of processing are, for example, processing that is performed by the units of the processing unit 140 as follows.

The biological information acquiring unit 143 acquires measurement information for a providing user, that is, a user targeted for processing. In this embodiment, the biological information acquiring unit 143 acquires the measurement information transmitted from the terminal device 600 of the user and received by the receiving unit 120, from the user information storage unit 115. The biological information acquiring unit 143 acquires measurement information on a user targeted for processing, from the user information storage unit 115, based on the user identifier of the user.

The biological information processing unit 146 performs predetermined filter processing on measurement information acquired by the biological information acquiring unit 143 (referred to as pre-processing information), thereby acquiring measurement information after the processing. The biological information processing unit 146 may also be said to perform dimensionality reduction (dimensionality compression) on the pre-processing information. The biological information processing unit 146 is configured, for example, to perform known singular spectrum analysis, as the predetermined filter processing. Accordingly, content information can be acquired using measurement information after the processing in which oscillating and trend components are extracted. Such filter processing can be performed, for example, using a numerical calculation library such as NumPy, for example.

The biological information processing unit 146 is configured to acquire measurement information after the processing, using results having degrees up to a predetermined highest degree obtained through singular spectrum analysis. The predetermined degree is, for example, 1 or higher and 10 or lower, and preferably 5 or higher and 10 or lower. In this embodiment, the biological information processing unit 146 is configured to acquire measurement information after the processing, using the results having degrees up to a highest degree of 10 obtained through singular spectrum analysis.

The method by which the biological information processing unit 146 performs dimensionality reduction is not limited to this. For example, the biological information processing unit 146 may be configured to perform dimensionality reduction of pre-processing information using an autoencoder using a convolutional neural network. The dimensionality reduction using an autoencoder can be configured using known methods. For example, the pre-processing information may be input to a probabilistic encoder to extract vectors indicating latent oscillating components and vectors indicating latent trend components, respectively.

For example, in the case in which measurement results in the form of ECG are used, the measurement information after the processing by the biological information processing unit 146 can be, for example, time-series information indicating sympathetic and parasympathetic activities in the providing user, respectively, as described later. That is to say, the measurement information can be information on a time period during which the providing user is likely to feel stressed or a time period during which the providing user is likely to be relaxed in a predetermined period of time. Examples of the predetermined period of time include, but are not limited to, the period during which a biological signal was measured.

The learning information acquiring unit 149 generates learning information, using a machine learning method. In this embodiment, for example, it is possible to use learning information (which may be referred to as first learning information) in which measurement information is input and used to output specifying information and learning information (which may be referred to as second learning information) in which the specifying information is input and used to output content information.

In this case, the specifying information is information for specifying a status of the providing user. The status of the providing user is, for example, information for specifying the rhythm, the condition, the health status, and the like of the body. The specifying information may be information indicating that the status of the providing user falls under a predetermined category (result of categorization into a predetermined category), or it may be a score. The specifying information may contain degree information indicating a degree to which the status of the providing user falls under a predetermined category. The specifying information is information acquired using the measurement information as will be described later, and may be said to be information based on the measurement information.

In this embodiment, the specifying information is constituted by, for example, whether or not the user is relaxed (an example of the predetermined category) and degree information for specifying the degree thereof. For example, information that can be expressed as "the user is relaxed and the degree thereof is 3 out of 5" or "the user is not relaxed (feeling stress) and the degree thereof is 5 out of 5" can be used as the specifying information. In the specifying information, there may be two or more categories of the user's status.

The machine learning method can be used as follows, for example. That is to say, two or more pairs of information that is input and information that is output are prepared in advance, and the two or more pairs of information are given to a module for configuring a learning model of machine learning to configure a learning model. That is to say, the learning information acquiring unit 149 generates first learning information using two or more pieces of training data each having input information containing at least measurement information and a categorization result regarding a physical condition of a user. Furthermore, the learning information acquiring unit 149 generates second learning information using two or more pieces of training data each having input information containing at least specifying information and content information. The learning model may also be said to be a classifier. There is no limitation on the machine learning method, and examples thereof include deep learning such as convolutional neural networks (CNNs), random forests, and SVR. For example, functions in various machine learning frameworks and various existing libraries, such as fastText, tinySVM, random forest, and TensorFlow, can be used for the machine learning.

In this case, the second learning information may be, for example, learning information configured to acquire content information from specifying information using an generative adversarial network (GAN). The second learning information may be a machine learning module configured such that a phrase is used as input to output content such as images, such as the known Stable Diffusion.

The learning information acquiring unit 149 accumulates the configured learning model as learning information in the learning information storage unit 111. Accordingly, the processing unit 140 can acquire the generated learning information from the learning information storage unit 111 in the case of using the learning information.

The learning information acquiring unit 149 may be configured to acquire learning information stored in another apparatus that is not the information processing apparatus 100, from that apparatus. For example, if the learning information stored in another apparatus is always used, the information processing apparatus 100 may not be provided with the learning information storage unit 111.

The content generating unit 161 generates content information using the measurement information. The content generating unit 161 generates the content information using learning information configured such that input information containing measurement information or information based on the measurement information is used to output output information.

In this embodiment, the content generating unit 161 uses measurement information and first learning information to generate specifying information, which is information based on the measurement information. The specifying information is acquired by the specifying information acquiring unit 162. That is to say, the specifying information acquiring unit 162 acquires specifying information for specifying a status of the providing user, using the measurement information. The content generating unit 161 then uses the specifying information and second learning information to generate content information.

The content accumulating unit 163 accumulates the content information generated by the content generating unit 161, in the storage unit 110. This enables the processing unit 140 to use the generated content information in other processing and the like. For example, if the processing unit 140 accepts an output request or the like from a terminal device 600 or the like, the processing unit 140 can output the content information to the terminal device 600 or the like.

The content accumulating unit 163 accumulates the content information, in the storage unit 110, in association with a provider identifier with which the providing user of the measurement information used to generate the content information can be identified. Accordingly, the corresponding content information can be specified for each providing user. Also, the providing user corresponding to the content information can be specified. In other words, the providing user (the providing user who provided the measurement information) from whom the content information was obtained can be specified.

The content accumulating unit 163 may be configured to accumulate the provider identifier in a location different from the storage unit 110. In this case, it is sufficient that the content information and the provider identifier corresponding thereto are associated with each other. For example, the content information and its identifier may be stored in the storage unit 110, and the identifier for identifying the content information and the provider identifier may be stored in another storage location in association with each other. This can save the storage space occupied by the storage of information in the other storage location. A token described later may be used as an identifier for identifying the content information.

The token recording unit 164 is configured to acquire a non-fungible token (hereinafter simply referred to as a "token") corresponding to the content information generated by the content generating unit 161. The token recording unit 164 records the acquired token in a predetermined storage location.

Furthermore, in this embodiment, the token recording unit 164 is configured to perform first recording processing that records the token in a predetermined manner in association with a provider identifier for identifying a providing user corresponding to the content information. Also, the token recording unit 164 is configured to perform second recording processing that records the token in a predetermined manner in association with an owner identifier for identifying an owning user of the content information. The recording in a predetermined manner means, for example, recording in a predetermined storage location. The storage location may be the same or may be different between the first recording processing and the second recording processing. That is to say, the predetermined manner in the first recording processing and the predetermined manner in the second recording processing may be the same or may be different.

In this embodiment, the predetermined storage location is, for example, the storage unit 110. That is to say, the token recording unit 164 accumulates the token, the provider identifier, and the owner identifier in the content information storage unit 117 in association with each other. That is to say, as described above, the token, the provider identifier, and the owner identifier are accumulated in the content information storage unit 117 in association with the content information.

Another storage location different from the storage unit 110 may be used as the predetermined storage location. For example, the content information and its identifier may be stored in the storage unit 110, and the identifier for identifying the content information, and the provider identifier and the like and the token may be stored in another storage location in association with each other. This can save the storage space occupied by the storage of information in the other storage location.

Examples of the other storage location may include a storage unit of an apparatus different from the information processing apparatus 100, other database, and the like. That is to say, for example, a blockchain infrastructure may be used as the predetermined storage location. The blockchain infrastructure may be of a so-called public type or a private type. The storage unit 110 may be used as a storage unit constituting the blockchain infrastructure.

Through such processing performed by the token recording unit 164, the provider identifier and the owner identifier associated with the token corresponding to the content information can be clearly identified. This makes it possible to easily specify the providing user corresponding to the content information and the owning user who owns the content information.

The first recording processing is performed, for example, at the time of the generation of the content information. It may be simultaneous with the generation of the content information, or there may be a time lag therefrom. For example, in the case of managing records related to tokens using a blockchain infrastructure, the first recording processing may be performed if so-called minting is performed for the content information. In conjunction with the first recording processing being performed, the second recording processing may be performed such that the provider identifier associated with a token is associated with the token also as an owner identifier. However, there is no limitation to this, and in conjunction with the first recording processing being performed for a token, the second recording processing may be performed that records a specific owner identifier in association with the token.

Furthermore, the second recording processing is performed, for example, in the case in which information indicating that the owning user of the content information has changed is acquired by the token recording unit 164. For example, the second recording processing may be performed in the case in which the trading information is acquired as follows.

That is to say, the content information can be used as an item to be sold and bought between users. It can also be said to be treated as an item to be transferred between users. For example, the token recording unit 164 is configured to perform the following processing in the case in which the content information is sold between users.

First, the token recording unit 164 acquires sales information on the sale. The sales information may be, for example, information acquired from an apparatus (which may be the information processing apparatus 100 or any other apparatus) that has performed processing related to the sale, or it may be based on information input or transmitted by the user or the like. The sales information may contain, for example, information with which a combination of a token corresponding to the sold content information and an identifier for identifying a user to whom the content information was sold (an identifier of the user who purchased the content information) can be specified. For example, the sales information may contain an owner identifier before the sale, an identifier for identifying the content information, and an identifier of the user to whom the content information was sold. It is sufficient that the sales information contains an identifier for identifying the content information and an identifier of the user to whom the content information was sold, in the case in which the content information is a unique item.

The token recording unit 164 then performs recording processing such that the token corresponding to the sold content information is associated with the owner identifier corresponding to the sales information. That is to say, an identifier of the user to whom the content information was sold is recorded as an owner identifier in association with the token.

Through this processing, the owner identifier recorded in association with the token is changed to an identifier after the sale, and the state is maintained in which the owner identifier corresponding to the owning user who owns the content information as a result of the sale is recorded in association with the token.

In the case of managing records related to tokens using a blockchain infrastructure as described above, such processing of the token recording unit 164 may be performed as processing using a smart contract function realized in the blockchain infrastructure.

The owner processing unit 165 performs predetermined owner processing using the owner identifier corresponding to the token, in the case in which a predetermined owner processing condition corresponding to the token is satisfied.

The predetermined owner processing condition is, for example, that a predetermined point in time has arrived, that new recording processing corresponding to the token has been performed by the token recording unit 164, that a predetermined request by the user or the like has been accepted, or the like. The predetermined point in time may be, for example, a predetermined date and time, or the time when a predetermined period of time has elapsed from a predetermined starting point. Examples of the predetermined starting point include, but are not limited to, the time when recording processing that associates the present owner identifier with the token is performed, the time when the content information is generated, the time that is set to be determined according to the provider identifier (e.g., a birthday associated with the provider, a date and time specified by the provider, etc.).

Examples of the predetermined owner processing include, but are not limited to, processing that determines whether or not to allow the provision of a predetermined service to the owning user, granting of authority, and transmission of information to the owning user.

For example, the owner processing unit 165 may be configured to be able to perform the following operation.

That is to say, in the case in which a request to provide a predetermined service for a token is accepted from a user, the owner processing unit 165 may determine that a predetermined owner processing condition corresponding to the token is satisfied. Then, as the predetermined owner processing, whether or not the user who made the provision request is an owning user corresponding to an owner identifier corresponding to the token may be determined, and whether or not to allow the provision of the service to the user may be further determined according to the result of the determination. Accordingly, it is possible to determine that the service can be provided to the owning user of the content information only in the case in which a provision request is received from the owning user.

Furthermore, for example, in the case in which a predetermined notification timing arrives, the owner processing unit 165 may determine that a predetermined owner processing condition corresponding to the token is satisfied. Then, as the predetermined owner processing, predetermined notification information may be transmitted to the contact corresponding to the owner identifier corresponding to the token. Accordingly, it is possible to transmit the notification information to an owning user of the content information.

The provider processing unit 167 performs predetermined provider processing using the provider identifier corresponding to the token, in the case in which a predetermined provider processing condition corresponding to the token is satisfied.

The predetermined provider processing condition is, for example, that a predetermined point in time has arrived, that new recording processing corresponding to the token has been performed by the token recording unit 164, that a predetermined request by the user or the like has been accepted, or the like. The predetermined point in time may be, for example, a predetermined date and time, or the time when a predetermined period of time has elapsed from a predetermined starting point. Examples of the predetermined starting point include, but are not limited to, the time when recording processing that associates the present owner identifier with the token is performed, the time when the content information is generated, the time that is set to be determined according to the provider identifier (e.g., a birthday associated with the provider, a date and time specified by the provider, etc.).

Examples of the predetermined provider processing include, but are not limited to, processing that determines whether or not to allow the provision of a predetermined service to the providing user, granting of authority to receive a predetermined service, and transmission of information to the providing user.

For example, the provider processing unit 167 may be configured to be able to perform the following operation.

That is to say, in the case in which first recording processing corresponding to a token is performed, the provider processing unit 167 may determine that a predetermined provider processing condition corresponding to the token is satisfied. Then, as the predetermined provider processing, processing that grants predetermined authority to the provider identifier may be performed. For example, the provider identifier and flag information indicating possession of the predetermined authority are accumulated in the storage unit 110 in association with each other. Accordingly, it is possible to confirm that the providing user possesses the predetermined authority. The processing that grants predetermined authority may be, for example, processing that transmits information to the providing user that serves as proof that the user possesses the predetermined authority. For example, a coupon code, an image of a two-dimensional code, or the like that enables the providing user to receive a predetermined service may be transmitted to the providing user, or may be provided so as to be acquired from the terminal device 600 that is used by the providing user or the like.

Furthermore, in the case in which new second recording processing corresponding to the token is performed by the token recording unit 164, the provider processing unit 167 may determine that a predetermined provider processing condition corresponding to the token is satisfied. In other words, in the case in which second recording processing is performed and an owner identifier corresponding to a token is changed to another owner identifier, it may be determined that a predetermined provider processing condition corresponding to the token is satisfied. In this case, the provider processing unit 167 may grant a predetermined reward to the providing user, using the provider identifier corresponding to the token. For example, the above-mentioned predetermined authority may be granted as the predetermined reward. For example, in the case in which the second recording processing is performed in conjunction with the sale of the content information, the right to receive a predetermined amount of reward or an amount of reward calculated in accordance with predetermined reward conditions based on the amount of sale or the amount of profit of the seller may be granted to the providing user. Such reward may be stipulated to be paid by the seller, by the new owning user, or by some other entities.

The predetermined owner processing condition and the predetermined provider processing condition corresponding to the tokens may be different for each token, or may be the same between the tokens.

The transmitting unit 170 transmits information to other devices constituting the information processing system 1 via a network. The transmitting unit 170 transmits information, for example, to the terminal device 600. In other words, the transmitting unit 170 outputs information, for example, to the terminal device 600.

Next, the configuration of a terminal device 600 will be described.

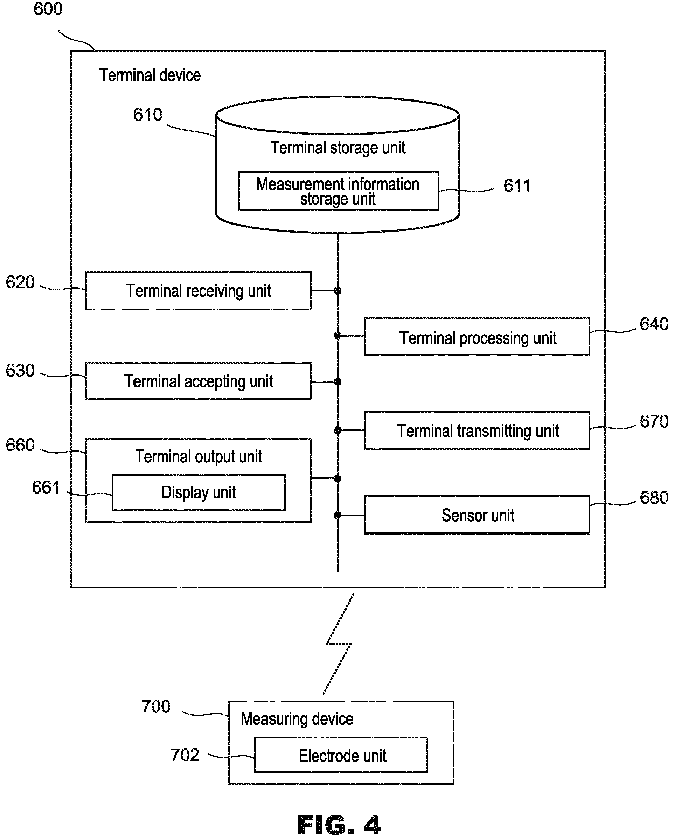

FIG. 4 is a block diagram of a terminal device 600 and a measuring device 700.

As shown in FIG. 4, the terminal device 600 includes a terminal storage unit 610, a terminal receiving unit 620, a terminal accepting unit 630, a terminal processing unit 640, a terminal output unit 660, a terminal transmitting unit 670, and a sensor unit 680. The terminal output unit 660 includes a display unit 661.

Various types of information are accumulated in the terminal storage unit 610. The terminal storage unit 610 includes a measurement information storage unit 611. For example, measurement information measured by the measuring device 700 is accumulated in the measurement information storage unit 611.

The terminal receiving unit 620 receives information transmitted from the information processing apparatus 100, the measuring device 700, and the like, via a network. The terminal receiving unit 620 accumulates the received information, for example, in terminal storage unit 610 such that the information can be acquired by the terminal processing unit 640 and the like.

The terminal accepting unit 630 accepts various input operations performed on the terminal device 600 by a user who uses the terminal device 600. The operations are performed using, for example, an unshown input device, but there is no limitation to this.

The terminal processing unit 640 performs various information processing operations using the units of the terminal device 600. For example, information transmitted from the information processing apparatus 100 is output by the terminal output unit 660 to the user. This allows the user to learn about the information.

The terminal output unit 660 outputs information by, for example, displaying it on the display unit 661 that is a display device. The method for outputting information is not limited to this, and may also be performed by outputting a sound or the like from a speaker or the like.

The terminal transmitting unit 670 transmits information acquired by the terminal processing unit 640 or the like, for example, via a network.

Examples of the sensor unit 680 include, but are not limited to, a microphone, an acceleration sensor, a barometric pressure sensor, and the like. The sensor unit 680 may be used to measure a biological signal of the user. The sensor unit 680 may include electrodes that can measure a biological signal of the user.

Next, an example of the operation of the information processing apparatus 100 according to this embodiment will be described.

In this embodiment, the information processing system 1 is typically used as follows. That is to say, the providing user measures his or her own biological signals while wearing the measuring device 700 over a predetermined period of time. The measurement information obtained from the measurement is made to be transferred to the terminal device 600. The providing user then transmits the measurement information stored in the terminal storage unit 610 from the terminal device 600 to the information processing apparatus 100. Accordingly, the information processing apparatus 100 acquires the specifying information and acquires the content information. Accordingly, a token corresponding to the content information as well as the provider identifier and the owner identifier are recorded in a predetermined manner. Thereafter, each time the owner of the content information changes, the second recording processing is performed. Each time a predetermined processing condition is satisfied, the owner processing or provider processing corresponding thereto is performed.

In the case in which such operations of the information processing system 1 are performed, the information processing apparatus 100 performs various operations as follows, for example. These operations are performed by the processing unit 140 executing control operations and the like while using the units.

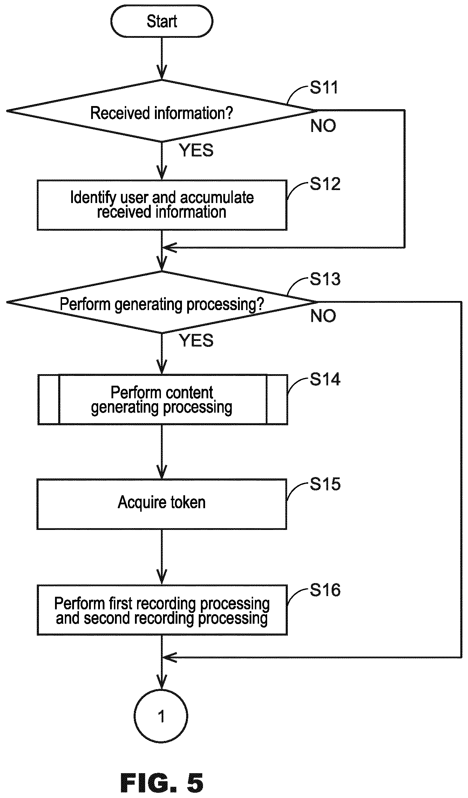

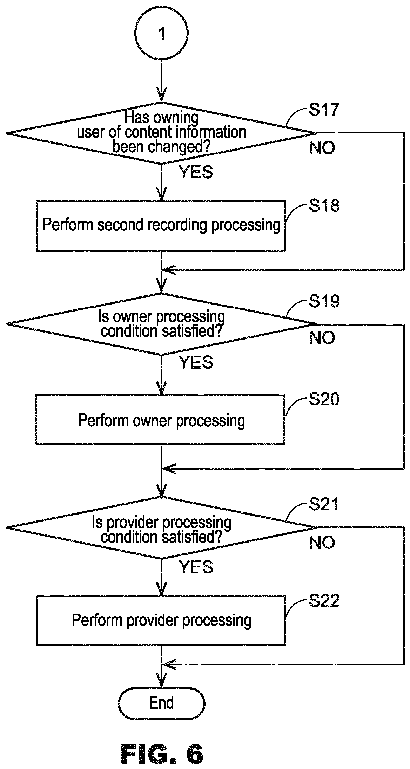

FIG. 5 is a first flowchart showing an example of an operation of the information processing apparatus 100. FIG. 6 is a second flowchart showing an example of an operation of the information processing apparatus 100.

(Step S11) The processing unit 140 determines whether or not the receiving unit 120 has received information transmitted from the terminal device 600 or the like. If it is determined that it has received the information, the procedure advances to step S12, or otherwise the procedure advances to step S13.

(Step S12) The processing unit 140 identifies the user based on the received information, and accumulates the received information in the user information storage unit 115 in association with the user identifier. For example, if measurement information is transmitted from the terminal device 600 in association with a user identifier, the processing unit 140 accumulates the received measurement information in the user information storage unit 115 in association with that user identifier.

(Step S13) The processing unit 140 determines whether or not a trigger to perform content information generating processing has occurred. In other words, the processing unit 140 determines whether or not a condition for starting content information generating processing has been satisfied. If it is determined that such a trigger has occurred, the procedure advances to step S14. Otherwise, the procedure advances to step S17.

In this case, for example, the above-mentioned trigger can be the provision of an instruction from the user (i.e., the providing user) through the terminal device 600 to perform content information generating processing. For example, the above-mentioned trigger can be the transmission of predetermined information corresponding to such an instruction through the terminal device 600, or the like. The trigger is not limited to these. For example, the above-mentioned trigger can be the arrival of a predetermined time, and in this case, the content information can be generated periodically. For example, the trigger can be the fulfillment of various conditions such as the new receipt of measurement information or other information.

(Step S14) The processing unit 140 causes the biological information processing unit 146 and the content generating unit 161 to perform content generating processing. The content generating processing will be described later. The content information is acquired and accumulated in the content information storage unit 117 through the content generating processing.

(Step S15) The processing unit 140 causes the token recording unit 164 to acquire a token corresponding to the content.

(Step S16) The processing unit 140 causes the token recording unit 164 to perform first recording processing and second recording processing. Accordingly, the provider identifier and the owner identifier are recorded in association with the token for the content information.

(Step S17) The processing unit 140 determines whether or not an owning user of any of the content information has been changed. For example, if sales information is accepted or if information regarding a change in the owning user is input by the user or the like, it is determined that the owning user of the corresponding content information has been changed. If it is determined that the owning user has been changed, the procedure advances to step S18, or otherwise the procedure advances to step S19.

(Step S18) The processing unit 140 causes the token recording unit 164 to perform second recording processing. Accordingly, the owner identifier after the change is associated with the token of the corresponding content information.

(Step S19) The processing unit 140 determines whether or not an owner processing condition is satisfied. If it is determined that the owner processing condition is satisfied, the procedure advances to step S20, or otherwise the procedure advances to step S21.

(Step S20) The processing unit 140 causes the owner processing unit 165 to perform owner processing.

(Step S21) The processing unit 140 determines whether or not a provider processing condition is satisfied. If it is determined that the provider processing condition is satisfied, the procedure advances to step S22, or otherwise the series of processing is ended.

(Step S22) The processing unit 140 causes the provider processing unit 167 to perform provider processing. Thereafter, the series of processing is ended.

It is sufficient that such processing is periodically repeated.

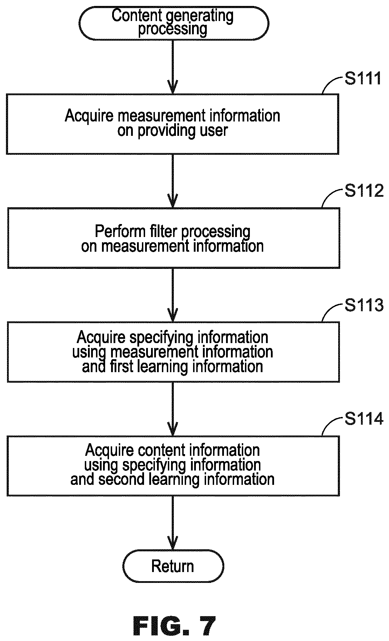

FIG. 7 is a flowchart showing an example of the content generating processing of the information processing apparatus 100.

(Step S111) The biological information processing unit 146 acquires measurement information on a providing user targeted for processing, from the user information storage unit 115, based on a user identifier of the providing user.

(Step S112) The biological information processing unit 146 performs filter processing on the measurement information.

(Step S113) The content generating unit 161 causes the specifying information acquiring unit 162 to acquire first learning information from the learning information storage unit 111 and to acquire specifying information using the measurement information and the first learning information. That is to say, the specifying information acquiring unit 162 applies the measurement information after the processing by the biological information processing unit 146 to the first learning information, thereby acquiring specifying information.

(Step S114) The content generating unit 161 acquires second learning information from the learning information storage unit 111, and acquires content information using the specifying information and the second learning information. That is to say, the content generating unit 161 applies the specifying information acquired by the specifying information acquiring unit 162 to the second learning information, thereby acquiring content information. The acquired content information is accumulated in the content information storage unit 117. Thereafter, the procedure returns to the processing in FIG. 5.

Next, specific examples of the acquisition of content information by the information processing apparatus 100 according to this embodiment will be described.

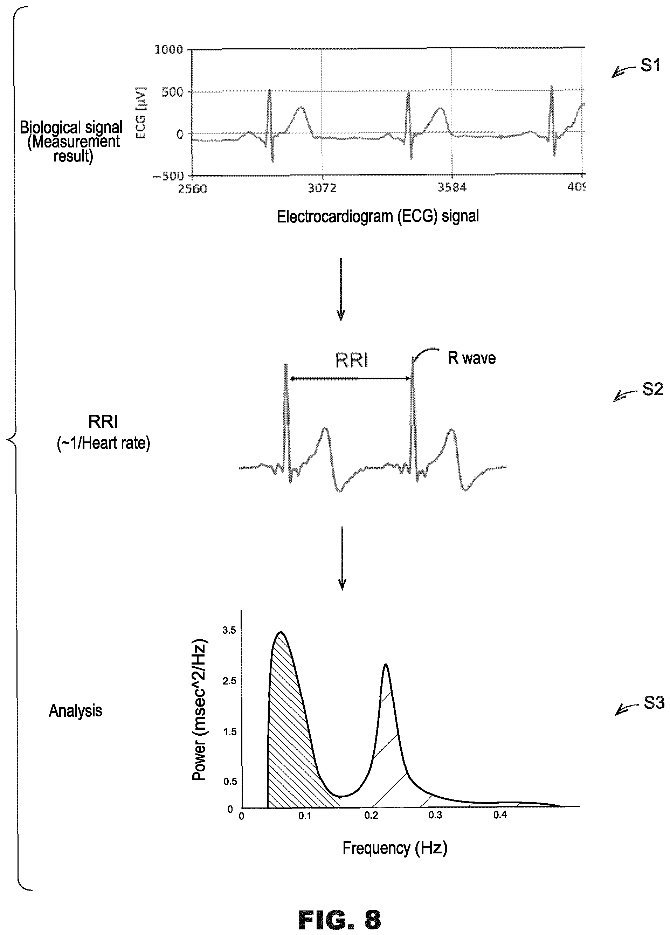

FIG. 8 is a diagram illustrating measurement information in a specific example of this embodiment.

In this specific example, the measurement information is time-series information acquired based on the information showing an ECG of a user as measurement results. The measurement information is information indicating the transition of LF and HF related to heartbeat variability of a user. The measurement information may be information on the transition of LF/HF (ratio of LF to HF).

LF and HF are values obtained by integrating the power spectrum obtained by frequency analysis of the transition of heartbeat intervals in the respective frequency domain bands (low-frequency band and high-frequency band). LF can be used as an indicator of the degree of activation of the user's sympathetic nervous system, and HF can be used as an indicator of the degree of activation of the user's parasympathetic nervous system. That is to say, LF, HF, and LF/HF can be used as information on the degree of activation of the user's autonomic nervous system.

As shown in the drawing, if the electrocardiogram signal for a predetermined measurement period (e.g., 5 minutes) is obtained as a measurement result (S1), the RRIs (heartbeat intervals: intervals between R waves) is obtained therefrom (S2). LF and HF can be obtained based on the power spectrum obtained by frequency analysis of the transition of RRIs over the predetermined measurement period (S3). LF and HF obtained for each predetermined measurement period can be summarized as measurement information.

Such acquisition of measurement information from the ECG may be performed, for example, by the terminal device 600, but it may also be performed by the measuring device 700 or by the information processing apparatus 100 that acquired the measurement results.

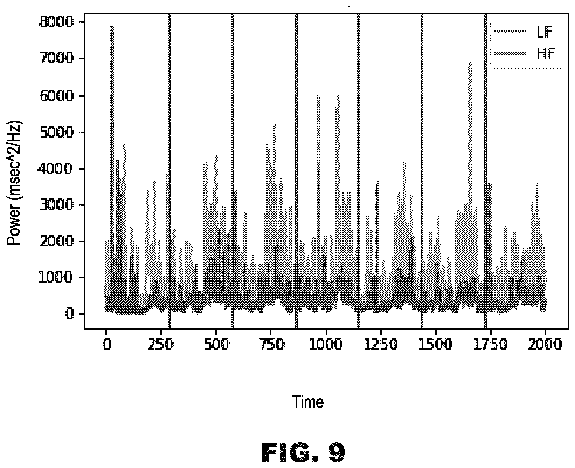

FIG. 9 is a diagram showing an example of measurement information before filter processing in a specific example of this embodiment.

The drawing shows measurement information indicating the transition of LF and HF over time, which is obtained as described above. In this drawing and the next drawing, for example, measurement information for approximately one week is shown. If such measurement information is filtered by the biological information processing unit 146, the result is as follows.

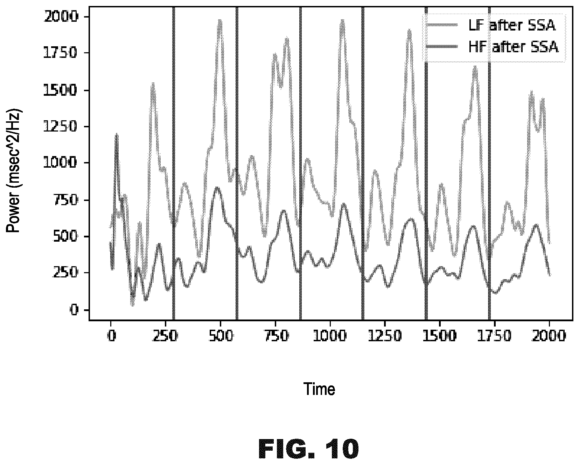

FIG. 10 is a diagram showing an example of measurement information after filter processing in a specific example of this embodiment.

The drawing shows measurement information after the processing obtained by restoring the measurement information, using the results having degrees up to a highest degree of 10 obtained through singular spectrum analysis, in contrast to the previous drawing. Such measurement information after the processing is information that well represents the transition of LF and HF of the user, with the principal components of the measurement information well extracted. By using such measurement information after the processing, it is possible to acquire specifying information that well represents the user's status.

In this specific example, the specifying information can be, for example, information for specifying a status in the intermediate representation system expressed as follows.

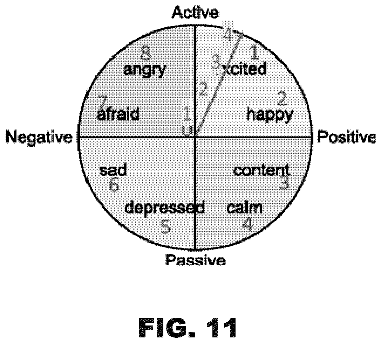

FIG. 11 is a diagram illustrating an intermediate representation system representing specifying information in a specific example of this embodiment.

The drawing shows a representation system consisting of two axes respectively indicating whether the user's mental status is positive or negative and whether the user's mental status is active or passive. The quadrants defined by the two axes show major categories of mental status, such as: exciteed; happy; content; calm; depressed; sad; afraid; and angry. As the specifying information, information specifying the coordinates in such a representation system can be used, and in this case, the category corresponding to the coordinates can be used as the specifying information. In the representation system, the further away from the origin, the stronger the elements of each of the two axes may be represented. In this case, the degree information that matches the corresponding category can be used as the specifying information.

In such an intermediate representation system, if the user's status can be represented as, for example, "the degree of being active is 3 out of 4 and the degree of being positive is 1 out of 4", information to the effect that, for example, "the user is excited and the degree thereof is 4 out of 4" can be acquired as the specifying information.

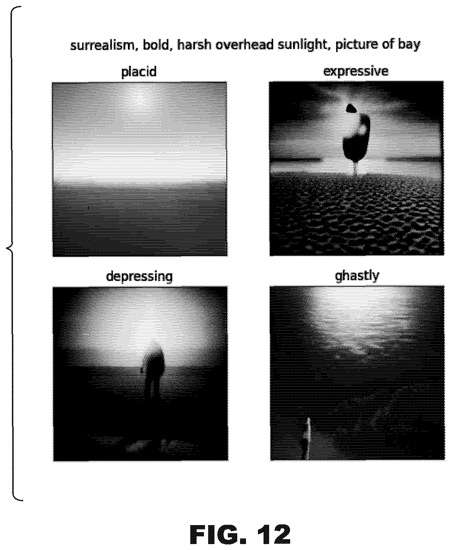

FIG. 12 is a diagram showing an example of the generation of content information in a specific example of this embodiment.

The drawing shows examples of the generation of images as content information for the cases in which four patterns of information "placid", "expressive", "depressing", and "ghastly" are obtained as the specifying information. In this case, in addition to the specifying information, a predetermined phrase (e.g., "surrealism, bold, harsh overhead sunightm, picture of day") is used as keywords indicating what kind of content is generated. If such a phrase is used, it may be changed or generated according to predefined conditions, for example, by each day or number of times it is generated. It is also possible to use a phrase that corresponds to the attributes of the providing user.

As explained above, the information processing apparatus 100 can generate content information from measurement information on a status of a living body of a providing user. Accordingly, it is possible to utilize measurement information. The content information is easily generated to correspond well to the status of the living body of the user, using learning information. In this embodiment, information on an owning user of the content information is reliably managed by recording the token in association with the owner identifier. Accordingly, it is possible to reliably perform owner processing regarding an owning user of the content information. In this embodiment, information on a providing user corresponding to the content information is reliably managed by recording the token in association with the provider identifier. Accordingly, it is possible to reliably perform provider processing regarding a providing user of content information.

If such information on an owner and the like corresponding to the token is managed, for example, using a blockchain infrastructure, it is possible to easily and reliably perform owner processing and provider processing using a smart contract function and the like. If authority such as rewards is granted to the providing user in predetermined cases as the provider processing, it is possible to effectively motivate the user to provide measurement information for generating content information.

The thus configured information processing system 1 can be used, for example, to construct an information distribution system 90 as follows.

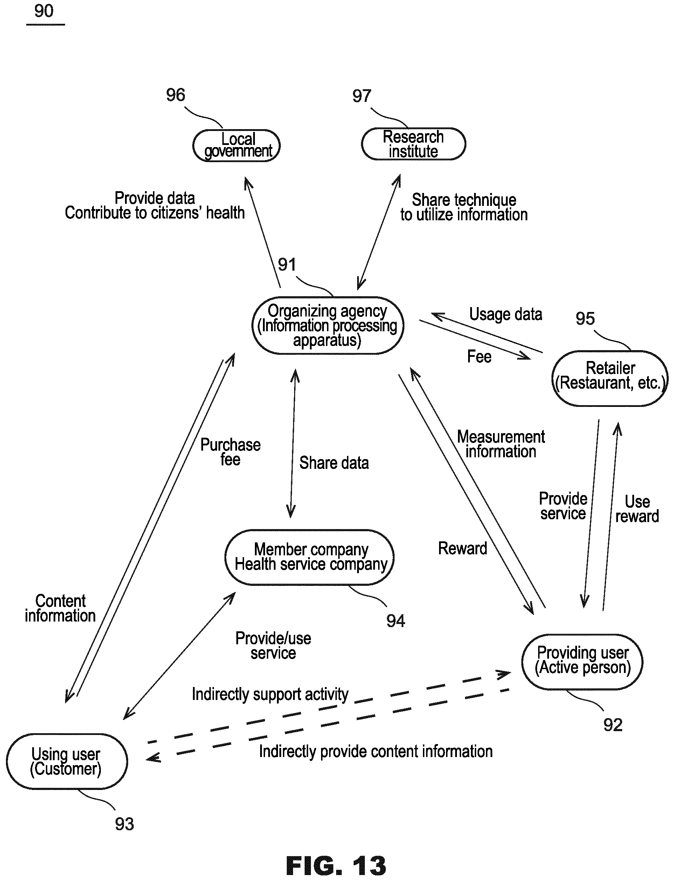

FIG. 13 is a diagram illustrating an example of the use of the information processing system 1.

The drawing roughly shows entities that may be involved in the information distribution system 90 and the relationships among the entities. The entities can include an organizing agency 91, a providing user 92, a using user 93, a member company 94, a retailer 95, a local government 96, and a research institute 97. In the drawing, the sale of generated content information and collection of purchase fees are shown as being performed by the information processing apparatus 100.

The organizing agency 91 prepares the information processing apparatus 100, sells the measuring devices 700 for using the information processing system 1, and provides applications for using the information processing system 1 on the terminal devices 600, for example.

The providing user 92 is, for example, an active person who creates new value for society. The providing user 92 transmits measurement information to the information processing apparatus 100. This generates content information and its token.

The using user 93 is, for example, a customer who can invest in health or various social initiatives. The using user 93 purchases desired content information and pays for the purchase. The using user 93 may designate an active person the using user wants to support and purchase his or her content information.

If the content information is purchased by the using user 93 in this manner, a reward is given to the providing user 92 from the purchase fee. Furthermore, a fee is given to the retailer 95 to provide services to the providing user 92. The providing user 92 can use the reward to receive services from the retailer 95. The data on the use of the services by the providing user 92 may be sent by the retailer 95 to the organizing agency 91 for utilization.

If such an information distribution system 90 is constructed, the providing user 92 can receive indirect support from the using user 93 to conduct his or her own activities.

The using user 93 may be able to receive services provided by the member company 94 by owning the content information. The measurement information of each providing user 91, the usage data of the retailer 95, the usage data of the member company 94 by the using user 93, and the like, which are obtained by the information distribution system 90, may be provided to the local government 96 to be used for the health of its citizens. In addition, the technique may be shared between the organizing agency 91 and the research institute 97 to utilize the information.

The storage unit 110 and the terminal storage unit 610 described above are preferably non-volatile recording media, but they may alternately be realized by volatile recording media. Information and the like acquired by each apparatus are stored in the corresponding units, but the procedure in which information and the like are stored is not limited to this. For example, information and the like may be stored via a recording medium, information and the like transmitted via a communication line or the like may be stored, or information and the like input via an input device may be stored.

Furthermore, the processing unit 140 and the terminal processing unit 640 described above may be typically realized by MPUs, memories, or the like. Typically, the processing procedure of the processing unit 140 and the terminal processing unit 640 is realized by software, and the software is stored in a recording medium such as a ROM. Note that the processing procedure may be realized by hardware (dedicated circuits).

Furthermore, the information that can be accepted by the accepting unit 130 or the terminal accepting unit 630 may be input by any input part such as a numeric keypad, a keyboard, a mouse, or a menu screen. The accepting unit 130 and the terminal accepting unit 630 may be realized by device drivers for an input part such as a numeric keypad or a keyboard, control software for a menu screen, or the like.

Furthermore, the receiving unit 120 and the terminal receiving unit 620 are typically realized by wireless or wired communication parts, but they may also be realized by broadcast receiving parts.

Furthermore, the transmitting unit 170 and the terminal transmitting unit 670 are typically realized by wireless or wired communication parts, but they may also be realized by broadcasting parts.

The information processing apparatus 100 may be constituted by a single server, multiple servers that operate in cooperation with each other, or a computer or other device built into other equipment. It will be appreciated that the servers may be so-called cloud servers, ASP servers, or the like, and there is no limitation on the type thereof.

The processing in this embodiment may be realized by software. The software may be distributed by software downloads or the like. Furthermore, the software may be distributed in a form where the software is stored in a recording medium such as an optical disk. The software that realizes the information processing apparatus 100 in this embodiment is the following sort of program. Specifically, this program is a program that is executed on a computer of the information processing apparatus 100, the program causing the computer of the information processing apparatus 100 capable of accessing a storage unit in which information is stored, to function as: a biological information acquiring unit that acquires measurement information on a status of a living body of a providing user; a content generating unit that generates content information using the measurement information; and a content accumulating unit that accumulates the content information generated by the content generating unit, in the storage unit.

### Others

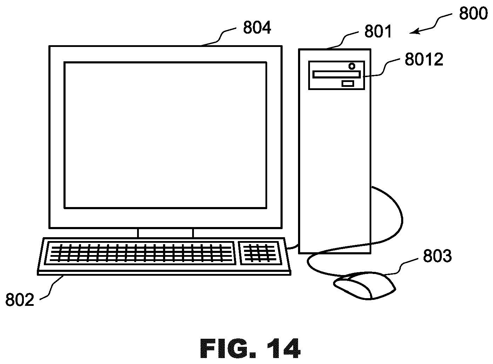

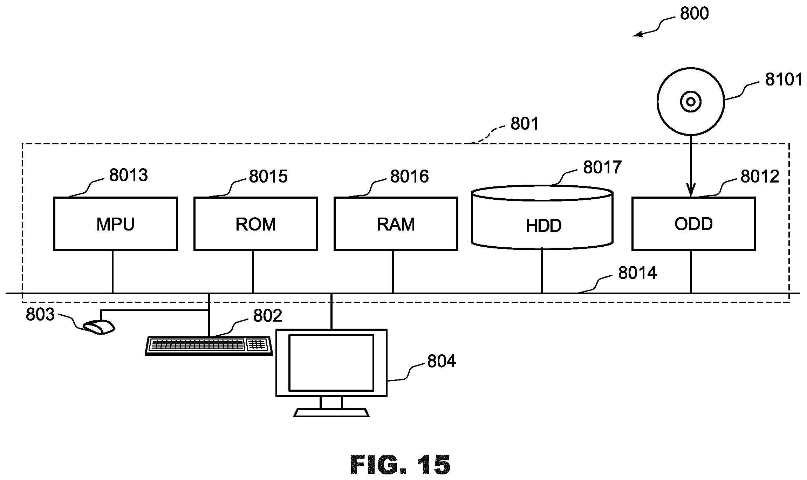

FIG. 14 is a schematic view of a computer system 800 in the foregoing embodiment. FIG. 15 is a block diagram of the computer system 800 in the embodiment.

These drawings show a configuration example of a computer that executes the programs described in this specification to realize the information processing apparatus and the like in the foregoing embodiment. The foregoing embodiment may be realized using computer hardware and a computer program executed thereon.

The computer system 800 includes a computer 801 including an optical disk drive, a keyboard 802, a mouse 803, and a monitor 804.

The computer 801 includes, in addition to the optical disk drive (ODD) 8012, an MPU 8013, a bus 8014 connected to the optical disk drive 8012 and the like, a ROM 8015 in which a program such as a boot up program is stored, a RAM 8016 that is connected to the MPU 8013 and is a memory in which a command of an application program is temporarily stored and a temporary storage area is provided, and a hard disk (HDD) 8017 in which programs such as an application program and a system program and data are stored. Although not shown, the computer 801 may further include a network card that provides connection to a LAN.

Each program for causing the computer system 800 to execute the functions of the information processing apparatus and the like in the foregoing embodiment may be stored in a optical disk 8101 that is inserted into the optical disk drive 8012, and be transmitted to the hard disk 8017. Alternatively, the program may be transmitted via a network (not shown) to the computer 801 and stored in the hard disk 8017. At the time of execution, the program is loaded into the RAM 8016. The program may be loaded from the optical disk 8101, or may be loaded directly from a network.

The program does not necessarily have to include, for example, an operating system (OS) or a third party program to cause the computer 801 to execute the functions of the information processing apparatus and the like in the foregoing embodiment. The program may only include a command portion to call an appropriate function (module) in a controlled mode and obtain desired results. The manner in which the computer system 800 operates is well known, and thus a detailed description thereof has been omitted.

It should be noted that, in the program, in a transmitting step of transmitting information, a receiving step of receiving information, or the like, processing that is performed only hardware such as processing that is performed by a modem or an interface card in the transmitting step (processing that can be performed only by hardware) is not included.

Furthermore, the computer that executes the program may be constituted by a single computer, or constituted by multiple computers. That is to say, centralized processing may be performed, or distributed processing may be performed.

Furthermore, in the foregoing embodiment, two or more constituent elements in one apparatus may be physically realized by one medium.

In the foregoing embodiment, each process (function) may be realized as centralized processing using a single apparatus (system), or may be realized as distributed processing using multiple apparatuses (in this case, the entire system constituted by multiple apparatuses that perform distributed processing may be regarded as one "apparatus").

Furthermore, in the foregoing embodiment, information transmission performed between constituent elements may be such that, for example, if two constituent elements for transmitting information are physically different from each other, the transmission is performed by one of the constituent elements outputting the information and the other constituent element accepting the information, or alternatively, if two constituent elements for transmitting information are physically the same, the transmission is performed by shifting from a processing phase corresponding to one of the constituent elements to a processing phase corresponding to the other constituent element.

Furthermore, in the foregoing embodiment, information related to the processing that is performed by each constituent element, for example, information that is to be accepted, acquired, selected, generated, transmitted, or received by each constituent element, information such as a threshold value, a numerical expression, or an address used by each constituent element in the processing and the like may be retained in an unshown recording medium temporarily or for a long period of time even if not specified in the description above. Furthermore, the information may be accumulated in the unshown recording medium by each constituent element or by an unshown accumulating unit. Furthermore, the information may be read from the unshown recording medium by each constituent element or by an unshown reading unit.

Furthermore, in the foregoing embodiment, if information used by each constituent element or the like, for example, information such as a threshold value, an address, or various setting values used by each constituent element in the processing may be changed by a user, the user may be or may not be allowed to change such information as appropriate even if not specified in the description above. If the user is allowed to change such information, the change may be realized by, for example, an unshown accepting unit that accepts a change instruction from the user and an unshown changing unit that changes information according to the change instruction. The unshown accepting unit may accept the change instruction, for example, by accepting information from an input device, by receiving information transmitted via a communication line, or by accepting information read from a predetermined recording medium.

The present invention is not limited to the embodiment set forth herein. Various modifications are possible within the scope of the invention.

An embodiment may be configured by combining constituent elements of the foregoing embodiment or modified examples as appropriate. For example, the configuration of the foregoing embodiment is not limited to those described above, and constituent elements of the foregoing embodiment or modified examples may be replaced by or combined with other constituent elements as appropriate. Some of the constituent elements or the functions may be omitted in the foregoing embodiment or modified examples.

In the foregoing embodiment, the content information is generated from the measurement information using the first learning information and the second learning information, but there is no limitation to this. For example, the specifying information acquiring unit may acquire the specifying information using the measurement information and the first learning information, and the content generating unit may acquire, as the content information, information selected based on the specifying information from among two or more pieces of candidate information of content information prepared in advance. The specifying information acquiring unit may acquire the specifying information using the first learning information, and the content generating unit may acquire input information corresponding to the specifying information, and acquire the content information using the acquired input information and the second learning information.

Furthermore, the content generating unit may be configured to acquire the content information, by applying the measurement information to learning information configured such that the measurement information is taken as input and the content information is taken as output.

Furthermore, the filter processing does not have to be performed on the measurement information .

### Industrial Applicability

As described above, the information processing apparatus according to the present invention makes it possible to generate content information from information on a status of a living body of a providing user, thus rendering this apparatus useful as an information processing apparatus and the like.

### List of Reference Numerals

- 1: Information processing system
- 100: Information processing apparatus
- 110: Storage unit
- 111: Learning information storage unit
- 115: User information storage unit
- 117: Content storage unit
- 120: Receiving unit
- 130: Accepting unit
- 140: Processing unit
- 143: Biological information acquiring unit
- 146: Biological information processing unit
- 149: Learning information acquiring unit
- 161: Content generating unit
- 162: Specifying information acquiring unit
- 163: Content accumulating unit
- 164: Token recording unit
- 165: Owner processing unit
- 167: Provider processing unit
- 170: Transmitting unit
- 600: Terminal device
- 610: Terminal storage unit
- 620: Terminal receiving unit
- 630: Terminal accepting unit
- 640: Terminal processing unit
- 660: Terminal output unit
- 661: Display unit
- 670: Terminal transmitting unit
- 680: Sensor unit
- 700: Measuring device
- 702: Electrode unit

## Claims

1. An information processing apparatus comprising:
a biological information acquiring unit that acquires measurement information on a status of a living body of a providing user;
a content generating unit that generates content information using the measurement information; and
a content accumulating unit that accumulates the content information generated by the content generating unit, in a storage unit in which information is stored.

2. The information processing apparatus according to claim 1, wherein the content generating unit includes a specifying information acquiring unit that acquires specifying information for specifying a status of the providing user, using the measurement information, and is configured to acquire the content information based on the specifying information.

3. The information processing apparatus according to claim 2, wherein the specifying information contains degree information indicating a degree to which the status of the providing user falls under a predetermined category.

4. The information processing apparatus according to claim 1, wherein the content generating unit generates the content information using learning information configured such that input information containing the measurement information or information based on the measurement information is used to output output information.

5. The information processing apparatus according to claim 1, further comprising:
a token recording unit that performs recording processing that records a non-fungible token corresponding to the content information generated by the content generating unit, in a predetermined manner in association with an owner identifier for identifying an owning user of the content information; and
an owner processing unit that performs predetermined owner processing using the owner identifier corresponding to the token in a case in which a predetermined processing condition corresponding to the token is satisfied.

6. The information processing apparatus according to claim 5, wherein in a case in which the token recording unit acquires sales information on a sale of the content information, the token recording unit performs the recording processing such that the token corresponding to the sold content information is associated with the owner identifier corresponding to the sales information.

7. The information processing apparatus according to claim 5 or 6,
wherein the token recording unit is configured to perform recording processing that records the token in a predetermined manner in association with a provider identifier for identifying a providing user corresponding to the content information, and
the information processing apparatus further comprises a provider processing unit that performs predetermined provider processing using the provider identifier corresponding to the token in a case in which a predetermined processing condition corresponding to the token is satisfied.

8. The information processing apparatus according to claim 7, wherein the provider processing unit grants a predetermined reward to the providing user using the provider identifier corresponding to the token, in a case in which the token recording unit performs recording processing that records the token newly in association with the owner identifier.

9. An information processing method performed using a storage unit in which information is stored, comprising:
a biological information acquiring step of acquiring measurement information on a status of a living body of a providing user;
a content generating step of generating content information using the measurement information; and
a content accumulating step of accumulating the content information generated in the content generating step, in the storage unit.

10. A program for causing a computer capable of accessing a storage unit in which information is stored, to function as:
a biological information acquiring unit that acquires measurement information on a status of a living body of a providing user;
a content generating unit that generates content information using the measurement information; and
a content accumulating unit that accumulates the content information generated by the content generating unit, in the storage unit.
